# EUROPÄISCHE PATENTSCHRIFT

(11) **EP 1 386 160 B1**
(45) Veröffentlichungstag und Bekanntmachung des Hinweises auf die Patenterteilung: **17.09.2008**
(21) Anmeldenummer: 02738051.8
(22) Anmeldetag: 10.05.2002
(51) Int. Cl.: G01N 33/543, G01N 33/80

(54) **VERFAHREN ZUM NACHWEIS VON BLUTZELL-ANTIGENEN UND GEGEN DIESE GERICHTETE ANTIKÖRPER**
METHOD FOR DETECTING BLOOD CELL ANTIGENS AND THE ANTIBODIES IN RESPONSE TO THE SAME
PROCEDE PERMETTANT DE DETECTER DES ANTIGENES DE GLOBULES SANGUINS ET DES ANTICORPS DIRIGES CONTRE CES ANTIGENES

(30) Priorität: 10.05.2001 DE 10122806
(43) Veröffentlichungstag der Anmeldung: 04.02.2004
(73) Patentinhaber: Kiesewetter, Holger, 13465 Berlin (DE); Salama, Abdulgabar, 13467 Berlin (DE)
(72) Erfinder: Kiesewetter, Holger, 13465 Berlin (DE); Salama, Abdulgabar, 13467 Berlin (DE)
(74) Vertreter: Luderschmidt, Schüler & Partner
(86) Internationale Anmeldenummer: PCT/EP2002/005140
(87) Internationale Veröffentlichungsnummer: WO 2002/090989

(56) Entgegenhaltungen:
- EP-A- 0 325 723
- DE-A- 4 228 395
- US-B1- 6 203 706
- LOELIGER C ET AL: "A RAPID AND SENSITIVE IMMUNOASSAY FOR ANTIBODIES AGAINST ALLOANTIGENS ON HUMAN PLATELET GLYCOPROTEINS (BIPA)" JOURNAL OF IMMUNOLOGICAL METHODS, ELSEVIER SCIENCE PUBLISHERS B.V.,AMSTERDAM, NL, Bd. 158, Nr. 2, 3. Februar 1992 (1992-02-03), Seiten 197-200, XP000371891 ISSN: 0022-1759
- STOCKELBERG D ET AL: "DETECTION OF PLATELET ANTIBODIES IN CHRONIC IDIOPATHIC THROMBOCYTOPENIC PURPURA (ITP) A COMPARATIVE STUDY USING FLOW CYTOMETRY, A WHOLE PLATELET ELISA, AND AN ANTIGEN CAPTURE ELISA" EUROPEAN JOURNAL OF HAEMATOLOGY, MUNSKGAARD, COPENHAGEN, DK, Bd. 56, Januar 1996 (1996-01), Seiten 72-77, XP000983326 ISSN: 0902-4441
- Produktbeschreibung zu Dynabeads M-280 Streptaviding (aus http://www.invitrogen.com/content/sfs/broc hures/2975_Streptavidin_screen.pdf)

## Beschreibung

Die vorliegende Erfindung betrifft ein Verfahren zum Nachweis von Antigenen auf Blutzellen bzw. den Nachweis von gegen diese Antigene gerichteten Antikörpern im Blut von Menschen. Dieses Verfahren wird in-vitro mit Hilfe von dem Patienten entnommenem Blut durchgeführt.

In der heutigen Diagnostik bestimmter Erkrankungen müssen im Blut der Patienten ausgewählte Antigene von roten und weißen Blutzellen (Erythrozyten bzw. Leukozyten) und von Blutplättchen oder auch spezifische Antikörper gegen diese Antigene nachgewiesen werden.

Blutzellantigene sind Oberflächenmarker (Merkmale) von roten Blutkörperchen (Erythrozyten), Blutplättchen (Thrombozyten) und weißen Blutkörperchen (Leukozyten). Meistens handelt es sich hierbei um Glykoproteine, Glykolipide oder Proteine. Eine Zusammenfassung hierzu findet sich z.B. in Transfusionsmedizin, Mueller-Eckhardt (Hgb), Springer-Verlag Berlin (1999), S. 137-200.

Die klinische Bedeutung dieser Blutzellantigene ergibt sich durch ihre Immunogenität, d. h. ihre Fähigkeit, in einem Individuum, das diese Antigene unter natürlichen Umständen nicht aufweist, die Bildung spezifischer Antikörper z.B. nach einer Transfusion, zu stimulieren.

Die Bildung solcher gegen Blutzell-Antigene gerichteten Antikörper kann weiterhin beispielsweise postnatal, wie es bei den Blutgruppenisoagglutininen (Anti-A, Anti-B) der Fall ist, oder durch die Übertragung allogener Zellen im Rahmen einer Schwangerschaft, einer Organtransplantantion oder einer Knochenmarkstransplantation induziert werden. Beispiele für entsprechende Antikörper sind Anti-D, Anti-K, anti-Fy usw. gegen Erythrozyten, Anti-HPA-1, Anti-HPA-2 gegen Thrombozyten, Anti-INA-1, Anti-INA-2 gegen Granulozyten oder Antikörper gegen HLA-Klasse I und Klasse II.

Darüber hinaus können Antikörper gegen eigene Antigene gebildet werden (Autoantikörper). Diese können zum vorzeitigen Abbau oder zur Zerstörung autologer Zellen führen, wie z. B. bei der autoimmunhämolytischen Anämie, der Autoimmunthrombozytopenie oder der Autoimmunneutropenie (Agranulozytose).

Alloantikörper gegen Erythrozyten können bei Feten und Neugeborenen unterschiedlich starke Hämolyse verursachen (Morbus haemolyticus neonatorum), vor allem Immunantikörper wie Anti-D und Anti-c.

Isoagglutinine und klinisch relevante Alloantikörper müssen vor jeder Bluttransfusion mit Erythrozyten berücksichtigt werden. Es dürfen in der Regel keine serologisch unverträglichen Erythrozyten transfundiert werden, z. B. rhesuspositive Erythrozytenkonzentrate, wenn bei dem Empfänger (rhesusnegativ) ein Alloantikörper der Spezifität Anti-D vorliegt. Daher müssen Antikörper gegen Erythrozyten vor Transfusionen identifiziert und bei einer Transfusion berücksichtigt werden. Ferner muss vor jeder Transfusion eine serologische Verträglichkeitsprobe (Kreuzprobe) mit allen vorgesehenen Konserven zur Transfusion durchgeführt werden.

Die Erfassung **von Antikörpern gegen erythrozytäre Antigene** erfolgt bisher routinemäßig durch die Inkubation von Serumproben gegen native ausgewählte Testerythrozyten (Antikörpersuche und ggf. Antikörperidentifizierung) bzw. vor einer Transfusion gegen Spendererythrozyten (Kreuzprobe). Die Unverträglichkeit zwischen Serum und Erythrozyten zeigt sich durch eine direkte oder indirekte Verklumpung der untersuchten Erythrozyten. Eine direkte Verklumpung der Erythrozyten wird z. B. durch die Isoagglutinine Anti-A und Anti-B verursacht. Eine indirekte Verklumpung wird z. B. induziert durch die Inkubation von Anti-D mit rhesuspositiven Testerythrozyten und anschließend durch die Gabe von Antihumanglobulinserum (indirekter Coombstest).

Die Nachteile der Verwendung von Testerythrozyten sind durch die Selektion, Seltenheit und Verfügbarkeit bestimmter Testerythrozyten und kurze Lagerungszeit der Zellen begründet. Es müssen regelmäßig ausgewählte Spender mit bekannten Antigenen zur Zellisolierung gesucht werden, und die isolierten Zellen sind nur für kurze Zeit haltbar.

Alle bisher beschriebenen Testmethoden beruhen praktisch auf der Verwendung solcher Zellen (siehe Transfusionsmedizin, Mueller-Eckhardt (Hgb), Springer-Verlag Berlin (1999), S. 587-596, und New diagnostic methods in oncology and hematology, Huhn (Hgb) Springer Verlag, Berlin, 1998, S.197-212).

Damit verbundene Nachteile sind daher:
- Zur Erkennung spezifischer Antikörper müssen mehrere ( in der Regel mehr als 11) Testerythrozyten (Testzellen) pro Testung verwendet werden, da Erythrozyten mit isolierten (einzelnen) Merkmalen nicht verfügbar sind (z. B. Zellen, die nur das Merkmal "D" tragen).
- In der Regel stehen Testerythrozyten mit seltenen Merkmalen zur Erkennung bestimmter Antikörper nicht zur Verfügung. Dadurch kann die Versorgung betroffener Patienten nicht immer gewährleistet werden.
- In der Regel stehen Testerythrozyten mit nicht vorhandenen Merkmalen, die normalerweise häufig vorkommen, zur Erkennung bestimmter Antikörper nicht zur Verfügung. Auch dadurch kann die Versorgung betroffener Patienten nicht immer gewährleistet werden.
- Die Selektion der entsprechenden Testzellen ist aufwendig.
- Da es sich um aus Menschen isoliertes biologisches Material handelt, ist ein Infektionsrisiko für das mit dem Material befasste klinische Personal, Ärzte etc. nicht immer ausgeschlossen.
- Die Zellen sind nur begrenzt lagerfähig.

Die klinische Bedeutung der **Antikörper gegen thrombozytäre Antigene** ist durch die Autoimmunthrombozytopenie (AITP), die neonatale Alloimmunthrombozytopenie (NAIT), die posttransfusionelle Purpura (PTP) und die Transfusion inkompatibler Thrombozyten reflektiert. In allen Fällen können die Antikörper eine primäre Hämostasestörung und eine Blutungsneigung bei den betroffenen Patienten verursachen.

Der Nachweis von Antikörpern gegen Thrombozyten ist bisher schwierig und kann nur in einzelnen Speziallabors durchgeführt werden. Ein einfacher und schneller Agglutinationsstest, wie z. B. bei Erythrozyten, ist durch die Beschaffenheit der Blutplättchen bisher nicht möglich. Von den bisher beschriebenen Methoden werden am häufigsten der **E**nzym-**l**inked-**I**mmunosorbent-assay (**ELISA**), der **P**lättchenadhäsions-**I**mmun**f**luoreszenztest (**PIFT**) und zur Spezifizierung der gebundenen Immunglobuline die Immunpräzipitation oder **MAIPA** (monoclonal antibody immobilization of platelet antigens)-Assay verwendet (siehe Transfusionsmedizin, Mueller-Eckhardt (Hgb), Springer-Verlag Berlin (1999)" S. 597-602, New diagnostic methods in oncology and hematology, Huhn (Hgb) Springer Verlag, Berlin, (1998)" S. 213-228, McMillan, Transfusion Medicine Reviews, Bd. IV, Nr. 2, pp 136-143 (1990)).

Die Nachteile dieser Methoden sind vielfältig:
- Alle Testmethoden sind arbeitsaufwendig, da typisierte Thrombozyten in der Regel nicht vorliegen, und die Tests nur von Experten durchgeführt werden können.
- Bei Patienten mit niedriger Thrombozytenzahl können häufig keine oder nicht genügend autologe Thrombozyten isoliert und untersucht werden, z. B im ELISA oder MAIPA.
- Die Spezifität (ELISA und PIFT) und die Sensitivität (MAIPA) sind limitiert, und dadurch können die Ergebnisse häufig nicht interpretiert werden.
- Desweiteren treffen im wesentlichen alle Nachteile zu, die oben auch für den Nachweis erythrozytärer Antigene beschrieben wurden.

Die klinische Bedeutung der **Antikörper gegen Neutrophilen-abhängige Antigene** ist durch die Autoimmunneutropenie, neonatale Alloimmunneutropenie und TRALI (Transfusions-assoziierte akute Lungen-Insuffizienz) gekennzeichnet. Die Erfassung dieser Antikörper ist noch schwieriger als die Erfassung thrombozytärer Antikörper. Die bisher bekannten Testmethoden sind weitgehend an Nachweismethoden von Antikörpern gegen Thrombozyten adaptiert (siehe Transfusionsmedizin, Mueller-Eckhardt (Hgb), Springer-Verlag Berlin (1999)" S. 603-609, New diagnostic methods in oncology and hematology, Huhn (Hgb) Springer Verlag, Berlin, (1998)" S. 228-235, Minchinton and Waters, British Journal of Haematology, 56, pp 521-528, (1984), McCullough and William, Transfusion Medicine Reviews, Bd. 1, Nr. 3, pp 150-160, (1987)).

Ferner sind Antikörper gegen Lymphozyten, vor allem T-Lymphozyten von größter Bedeutung in der Transplantationsimmunologie. Diese Antikörper können eine akute oder verzögerte Abstoßung der Transplantate (Knochenmark, Herz, Lunge, Niere und andere Organe) bewirken. Daher muss vor Durchführung von Knochenmarks- und Nierentransplantationen das Vorhandensein lymphozytärer Antikörper gegen Spenderorgane ausgeschlossen werden.

Weiterhin können Lymphozytenantigene (HLA-Antigene) im Falle von Schwangerschaften und Transfusionen zur Bildung von spezifischen Antikörpern gegen HLA-Merkmale führen. Das etwaige Vorhandensein solcher Antikörper muss deswegen bei Transplantationen und bei Transfusionen von Thrombozyten und ggf. Leukozyten berücksichtigt werden.

Die klassische Nachweismethode von HLA-Antikörpern ist bisher auf den lymphozytotoxischen Test beschränkt. Mit diesem Test können allerdings nur komplementaktivierende Antikörper bestimmt werden. Die neuerdings verwendete ELISA-Technik ist extrem schwierig und wird nur in einzelnen Speziallabors durchgeführt (siehe Transfusionsmedizin, Mueller-Eckhardt (Hgb), Springer-Verlag Berlin (1999)" S. 611-617, Zachary et al. Transplantation, Bd. 60, Nr. 12, pp 1600-1606 (1995), Lubenko und Rodi, Transfusion, Bd. 38, pp 41-44 (1998))

Zusammenfassend beruhen alle Testprinzipien zur Erfassung von Antikörpern gegen Blutzellen auf der Verwendung von ausgewählten biologischen und meist nativen Testzellen, die von ausgewählten Individuen gewonnen und zur Verfügung gestellt werden.

Ein relativ schneller und einfacher Test ist nur für die Erkennung von gewöhnlichen Antikörpern gegen Erythrozyten möglich, z. B. der Nachweis eines Anti-D-Antikörpers.

Die Suche nach Alternativen blieb bisher ohne Erfolg.

Obwohl die Gene der meisten Blutgruppensysteme (Erythrozyten, Thrombozyten und Leukozyten) inzwischen kloniert und zum Teil sequenziert wurden, gelingt der Nachweis von Antikörpern durch die Verwendung von rekombinanten Antigenen und Peptiden, wenn überhaupt, nur in Einzelfällen (Bowditch et al., Blood, Bd. 88, Nr. 12, pp 4579-4584 (1996), Peterson et al., Blood, Bd. 92, Nr. 6, pp 2053-2063 (1998), Yazdanbakhsh, Transfusion Medicine Reviews, Bd. 15, Nr. 1, pp 53-66, (2001)).

Auch der oben genannte MAIPA-Test, bei dem Antikörper auf Mikrotiterplatten zur Durchführung eines indirekten ELISA-Tests immobilisiert werden, bietet noch immer enorme praktische Handhabungsprobleme. So dauert dieser Test ca. 7-8 Stunden, und die Testergebnisse sind oft nicht eindeutig. Außerdem können die beschichteten Mikrotiterplatten nicht ausreichend lange aufbewahrt werden, so dass in der Praxis alle entsprechenden Lösungen im Labor frisch aufbereitet werden müssen.

Von Meyer et al., THE LANCET Bd. 354, Nr. 9189, pp 1525-1526 (1999) wurde ein Testsystem beschrieben, mit Hilfe dessen es gelang, Antikörper gegen den Komplex aus Plättchenfaktor 4 und Heparin nachzuweisen, indem mit Antigenen beschichtete Beads in einem Teilchen-Agglutinationstest mit Serumproben von Patienten versetzt wurden. Agglutinierte Proben wurden durch einen Gelkartentest (siehe z.B. Salama und Mueller-Eckhard in: Transfusionsmedizin, Mueller-Eckhardt (Hgb), Springer-Verlag Berlin (1999), Seite 587-617) nachgewiesen. Trotz angestrengtester Bemühungen gelang es den Erfindern jedoch nicht, mit Hilfe dieses relativ schnell und leicht durchzuführenden Tests andere Blutzell-Antigene oder auch gegen diese gerichtete Antikörper nachzuweisen.

Trotz des sich aus dem vorstehend Genannten zwangsläufig ergebenden extrem hohen Bedarfs nach einem Testsystem für den Nachweis von Antikörpern gegen ausgewählte Blutzell-Antigene, das die genannten Nachteile überwindet, konnte ein solches bis zu vorliegender Erfindung nicht zur Verfügung gestellt werden.

In der US 6,203,706 werden Verfahren aufgezeigt, mit deren Hilfe Blutzell-Antigene bzw. Antikörper in z.B. Blutproben nachgewiesen werden sollen. Es hat sich jedoch gezeigt, dass die entsprechenden Verfahren, bei denen Antigene direkt aufBeads gebunden werden, nur selten funktionieren.

Angesichts des genannten Stands der Technik lag der vorliegenden Erfindung daher die Aufgabe zugrunde, ein Verfahren zur Verfügung zu stellen, mit dem Antikörper gegen ausgewählte Blutzell-Antigene oder Blutzell-Antigene selbst spezifisch nachgewiesen werden können. Dieses Verfahren soll einfach und schnell in der Handhabung und sicher und kostengünstig in der Durchführung sein. Weiterhin soll das Verfahren in vitro an Proben durchgeführt werden können, die aus lebenden Organismen gewonnen werden können.

Gelöst wird diese sowie weitere, nicht explizit genannte Aufgaben, die jedoch aus den hierin einleitend diskutierten Zusammenhängen ohne weiteres ableitbar oder erschließbar sind, durch ein Verfahren mit allen Merkmalen des Patentansprüchs 1. Zweckmäßige Abwandlungen des erfindungsgemäßen Verfahrens werden in den rückbezogenen Unteransprüchen unter Schutz gestellt.

### VARIANTE 1:

Dadurch dass man bei einem in vitro-Verfahren
(i) ein Mikrokügelchen ("Bead"), die einer Durchmesser von 2-4 µm und eine Dichte von 1,1-1,8g/cm³ aufweisen, mit einem gegen das nachzuweisende spezifische Blutzellantigen gerichteten, nicht-humanen Antikörper beschichtet,
(ii) zugehörige, spezifische, ausgewählte Blutzell-Antigene, die gegebenenfalls durch Solubilisierung von im Stande der Technik an sich schon bekannten standardisierten Blutzellen oder auch durch rekombinante oder proteinchemische Methoden erhalten werden können, mit den aus (i) erhaltenen beschichteten Beads mischt,
(iii) die durch Bindung des ausgewählten Antigens an den in (i) an das Bead gebundenen, nicht humanen Antikörper mit Antigen beschichteten Beads mit einer Serumprobe eines zu untersuchenden Patienten mischt, und
(iv) den gegebenenfalls an den am Bead gebundenen Antigen gebundenen, aus der Probe stammende Blutzell-Antigen-Antikörper des Patienten mit Hilfe eines spezifischen anti-human-Antikörpertests durch Agglutination in einer Gelkarte nachweist,
gelingt es auf nicht ohne weiteres vorhersehbare Weise ein Verfahren zum Nachweis von Antikörpern gegen Blutzell-Antigene in einer Probe zur Verfügung zu stellen, welches diesen Nachweis auf einfache Art und Weise ermöglicht. Dabei weist dieses Verfahren die oben genannten Vorteile gegenüber dem Stand der Technik auf.

### VARIANTE 2:

Eine nur leichte Abwandlung des erfindungsgemäßen Verfahrens zum Nachweis von Antikörpern gegen Blutzell-Antigene bedeutet es, wenn man
(i) Beads, die einer Durchmesser von 2-4 µm und eine Dichte von 1,1-1,8g/cm³ aufweisen, mit nicht anti-humanen, Spezies-spezifischen Antikörpern beschichtet,
(ii) Standardzellen solubilisiert bzw. gentechnisch oder proteinchemisch gewonnene Blutzell-Antigene in Lösung bringt,
(iii) das aus (ii) erhaltene Solubilisat bzw. die Lösung von gentechnisch oder proteinchemisch gewonnenen Blutzell-Antigenen mit spezifischen, gegen das nachzuweisende Antigen gerichteten und aus der in (i) definierten Spezies stammenden Antikörpern inkubiert,
(iv) die in (iii) gebildeten Komplexe mit den Beads aus (i) durch Zentrifugation isoliert, und
(v) direkt oder später gegen aus Patienten stammendes Antikörper-haltiges Serum im Agglutinationstest untersucht.

### VARIANTE 3:

Weiterhin gelingt es dadurch, dass man bei einem in vitro-Verfahren
(i) ein Mikrokügelchen ("Bead"), die einer Durchmesser von 2-4 µm und eine Dichte von 1,1-1,8g/cm³ aufweisen, mit einem gegen das nachzuweisende spezifische Blutzellantigen gerichteten Antikörper beschichtet,
(ii) Blutproben des zu untersuchenden Patienten mit den darin enthaltenen Blutzellen einer Behandlung unterwirft, durch die die in der Membran der Blutzellen enthaltenen Antigene solubilisiert werden, und gegebenenfalls eine Antigen-angereicherte Fraktion herstellt,
(iii) die aus (i) erhaltenen beschichteten Beads mit der aus (ii) erhaltenen Probe mischt,
(iv) das gegebenenfalls an den am Bead gebundenen Antikörper gebundene, aus der Probe stammende Blutzell-Antigen mit Hilfe eines spezifischen Antikörpertests durch Agglutination in einer Gelkarte nachweist,

### VARIANTE 4:

Weiterhin ist es möglich, Blutantigene in Proben nachzuweisen, indem man
(i) Beads, die einer Durchmesser von 2-4 µm und eine Dichte von 1,1-1,8g/cm³ aufweisen, mit nicht anti-humanen, Spezies-spezifischen Antikörpern beschichtet,
(ii) Blutzellen aus zu untersuchenden Patienten solubilisiert,
(iii) das aus (ii) erhaltene Solubilisat mit spezifischen, gegen das nachzuweisende Antigen gerichteten und aus der in (i) definierten Spezies stammenden Antikörpern inkubiert,
(iv) die in (iii) gebildeten Komplexe mit den Beads aus (i) durch Zentrifugation isoliert, und
(v) die Probe im Agglutinationstest untersucht.

Insbesondere sind die erfindungsgemäßen Verfahren:
- einfach und schnell in der Handhabung und
- sicher und kostengünstig in der Durchführung,

Während zur Durchführung der bei den bisher im Stand der Technik beschriebenen Systeme bisher praktisch immer die Verwendung bzw. die zeitnahe Aufbereitung nativer Testzellen erforderlich war, kann das erfindungsgemäße Verfahren hierauf verzichten. Insbesondere bieten die fertig beschichteten, erfindungsgemäß verwendbaren Beads eine wesentlich höhere Lagerungsfähigkeit als native Testzellen. Außerdem können diese beschichteten Beads theoretisch in unbegrenztem Maße zur Verfügung gestellt und für den Bedarfsfall vorgehalten werden, so dass sich auch hieraus eine wesentliche Vereinfachung ergibt.

Essentiell für vorliegende Erfindung ist es, dass der Nachweis von Blutzell-Antigenen bzw. hier gegen gerichteten Antikörpern durchgeführt wird, in dem die nativen Antigene über die gegen sie gerichteten, monoklonalen Antikörper aus einer Probe gefischt werden. Nur mit dieser, gegenüber dem Stand der Technik wesentlichen Verfahrensabwandlung gelingt es, die oben genannte technische Aufgabe so leicht und sicher mit geringem Aufwand zu lösen.

Erfindungsgemäß verwendbare, fertig beschichtete Beads sind in Variante 1 die aus Stufe (ii) erhaltenen Beads, die in Variante 2 aus Stufe (iv) erhaltenen Beads, die in Variante 3 aus Stufe (i) erhaltenen Beads, sowie die in Variante 4 aus Stufe (i) erhaltenen Beads.

Diese Beads sind lange haltbar, wesentlich länger, als dies bei nativen Testzellen der Fall ist.

Da das biologische Ausgangsmaterial in extra darauf ausgerichteten Speziallabors aufbereitet werden kann, wird auch das biologische Infektionsrisiko für das befasste Personal verringert.

Erfindungsgemäß werden Blutzellantigene oder dagegen gerichtete Antikörper nachgewiesen. Zur Zeit sind mehr als 700 unterschiedliche erythrozytäre Antigene beschrieben. Von anderen Blutzellen sind noch weit mehr Antigene beschrieben. Alle diese Antigene besitzen oder können klinische Relevanz besitzen. Es ist daher für den Fachmann klar, dass mit der vorliegenden Erfindung alle diese Antigene prinzipiell schnell und sicher nachgewiesen werden können.

Eine Zusammenstellung solcher Blutzell-Antigene findet sich beispielsweise in Issitt, P. und Anstee, D. (Hgb.): APPLIED BLOOD GROUP SEROLOGY, Montgomery Scientific Publications, Durham, North Carolina, USA. Besonders wichtige Blutgruppenantigene, die mit Hilfe der vorliegenden Erfindung bevorzugt nachgewiesen werden, sind: AB0; C, C^{w}, c, D, E, e, K, k, Fy (a), Fy (b), Jk (a), Jk (b), S, s, M, N, P (1), Le (a), Le (b).

Die ebenfalls benötigten Antikörper sind bereits in großem Maße verfügbar. Beispiele für solche Antikörper sind: Anti-Jka (Fa. DiaClon), Anti-Jkb (Fa. DiaClon), Anti-Lea (DiaClon), Anti-Leb (DiaClon), Rh-Testseren der Firma BIOLITH DIAGNOSTIKA, Hann. Münden, Deutschland (Anti-D, Anti-C, Anti-c, Anti-C^{w}, Anti-E, Anti-e, Testseren des MNSs-Systems (Anti-M, Anti-N, Anti-S) oder auch die Seren der Fa. Dade (z.B. Anti-K₁). Insgesamt werden weltweit mehr als 600 unterschiedliche monoklonale Antikörper gegen Blutzell-Antigene kommerziell angeboten.

In Fällen, wo solche Antikörper nicht verfügbar sind, können diese entweder polyklonal oder monoklonal hergestellt werden. Methoden zur Herstellung von Antikörpern sind dem Fachmann gut bekannt.

Zur Durchführung der Solubilisierung der Blutzellen bzw. derer Antigene können die Protokolle angewendet werden, die auch in den im Stand der Technik bereits beschriebenen Verfahren verwendet werden. Wie an anderem Ort bereits ausgeführt, liegt ein ganz wesentlicher Vorteil der vorliegenden Erfindung darin, dass aufgrund der wesentlich erhöhten Haltbarkeit der beschichteten Beads diese Protokolle in dafür ausgerichteten Labors in großem Maßstab erfolgen können.

Zur Herstellung der erfindungsgemäß verwendbaren beschichteten Beads müssen die spezifischen Antikörper an die Beads gebunden werden.

Bevorzugt wird diese Bindung über den Streptavidin bzw. Avidin/Biotin Komplex vermittelt, indem biotinylierte Antikörper an mit Streptavidin bzw. Avidin beschichtete Mikrocarrier gebunden werden. Die entsprechenden Methoden sind dem Fachmann gut bekannt, und werden ausführlich in der US-Patentschrift 6,203,706 beschrieben.

Insbesondere ist es bevorzugt, wenn ein gegen das nachzuweisende Antigen gerichteter Antikörper, der biotinyliert ist, zunächst mit den nativen Zellen (Testzellen) reagiert, dann die Zellen solubilisiert werden, und der gebildete Komplex aus Antigen und biotinyliertem Antikörper mit Streptavidin oder mit Avidin beschichteten Beads (Microcarrier) aus dem Reaktionsansatz entfernt wird.

Dieser gebildete Komplex kann dann zweierlei verwendet werden:
1.) Soll das Vorhandensein eines gegen das Antigen gerichteten Antikörpers im Serum eines Patienten nachgewiesen werden, so kann der gebildete Komplex direkt mit dem Serum inkubiert werden. Eventuell vorhandene Antikörper reagieren dann mit dem Antigen und binden an den Komplex. Da diese nachzuweisenden Antikörper humanen Ursprungs sind, kann die Bindung der nachzuweisenden Antikörper nach Abtrennung des Komplexes aus dem Testansatz mittels eines anti-human Antikörper Antikörpers unter Verwendung der Gelkartenmethode erfolgen.
   Eine Aufzählung Methoden folgt: Standard-Antihumanglobulintest, Kartentest (Y. Lapierre et al., (1990), Transfusion 30(2):109-113), ELISA (Nachweis auf Mikrotiterplatte), Mikrochip, Kapillardiffusion, Durchflusszytometrie, Radioimmunoassay (RIA; z.B. Iod¹²⁵ Markierung der Beads) usw.
2.) Soll der direkte Nachweis des Antigens erfolgen, so kann direkt die Bindung eines Zweitantikörpers an den oben genannten Komplex aus Antigen und biotinyliertem Antikörper und mit Streptavidin oder mit Avidin beschichteten Beads mittels der gleichen Methoden erfolgen. Hierzu wird der Zweitantikörper erneut über einen spezies-spezifischen Antikörper mithilfe der Gelkartenmethode nachgewiesen, wobei der spezies-spezifische Antikörper gegen die Spezies gerichtet sein muss, aus der der Zweitantikörper stammt.

Besonders bevorzugte Varianten des erfindungsgemäßen Nachweissystems für Blutzellantigene bzw. gegen diese gerichtete Antikörper aus Serenproben umfassen daher:
Ein in vitro-Verfahren zum Nachweis von Antikörpern gegen Blutzell-Antigene in einer Serumprobe eines Patienten, bei dem man
   (i) biotinylierte, gegen das nachzuweisende Antigen gerichtete nicht-humane Antikörper in Kontakt bringt mit humanen Testzellen, die die entsprechenden, nativen Blutzell-Antigene tragen,
   (ii) den aus (i) gebildeten Komplex Bedingungen unterwirft, unter denen die Blutzellen solubilisieren,
   (iii) den Komplex aus Antigen und biotinyliertem Antikörper mit Hilfe von mit Streptavidin oder mit Avidin beschichteten Mikropartikeln aus der Probe entfernt,
   (iv) den in (iii) gebildeten Komplex mit einer Serumprobe eines zu untersuchenden Patienten in Kontakt bringt, und
   (v) die im Falle des Vorhandenseins von spezifischen Antikörpern in der Serumprobe gebildeten Komplexe aus biotinyliertem Antikörper, Antigen und humanem Antikörper aus dem Serum nach Abtrennung des Komplexes aus der Probe mit einem anti-human Antikörper Test durch Agglutination in einer Gelkarte nachweist.
   sowie
Ein in vitro-Verfahren zum Nachweis von ausgewählten Blutzell-Antigenen, bei dem man
   (i) biotinylierte, gegen das nachzuweisende Antigen gerichtete Antikörper in Kontakt bringt mit humanen Testzellen, die die entsprechenden, nativen Blutzell-Antigene tragen,
   (ii) den aus (i) gebildeten Komplex Bedingungen unterwirft, unter denen die Blutzellen solubilisieren,
   (iii) den Komplex aus Antigen und biotinyliertem Antikörper mit Hilfe von mit Streptavidin oder mit Avidin beschichteten Mikropartikeln aus der Probe entfernt,
   (iv) den in (iii) gebildeten Komplex mit einem gegen das Antigen gerichteten, bevorzugt markierten Zweitantikörper in Kontakt bringt, und
   (v) die gebildeten Komplexe aus biotinyliertem Antikörper, Antigen und Zweitantikörper nach Abtrennung des Komplexes aus der Probe durch Agglutination in einer Gelkarte nachweist.

Auch eine chemische Bindung des Antikörpers an die Beads kommt in Frage.

Als Beads werden für die Zwecke der vorliegenden Erfindung meist kugelförmige Microcarrier aus unterschiedlichen Materialien verstanden.

Bevorzugt werden hierbei ferromagnetische Streptavidin-Partikel (z.B. Dynabeads M280 Streptavidin) eingesetzt.

Die physikalischen Eigenschaften dieser Partikel sind wie folgt:
Durchmesser: 2-4 mm
Dichte:1,1-1,8 g/cm3
Oberfläche: 4-8 m2/g
Magnetisches Massenmoment: 100 ± 25x 10-6 m3/kg

Die Suspension enthält bevorzugt 10 mg/ml Partikel, entsprechend 6,7 x 10⁸ /ml

Die Bindungseigenschaften der Partikel sind bevorzugt wie folgt:
1 mg Dynabeads M280 Streptavidin können 5-10 mg eines biotinylierten Antikörpers binden (100 % Saturation des kovalent an der polystyrene Oberfläche gebundenen Streptavidin)

### Vorteile dieser Partikel sind:

Durch die paramagnetischen Eigenschaften eignen sich diese Partikel zur Isolierung von Biotin-Antikörper-Antigen-Komplexen aus einem heterogenen Gemisch, wie z.B. Zelllysat, durch einfache Anwendung eines Magneten (Magnetic Particle Concentrator).

Diese Partikel weisen aber folgende Nachteile auf:
- Es sind keine farbfluoreszenten Partikel vorhanden.
- Die Untersuchung in der Durchflußzytometrie ist ausgeschlossen.

Besonders bevorzugt erfindungsgemäß verwendbar sind daher Rot-fluoreszierende High-Density Streptavidin Polystyrene Partikel, die gut bekannt sind und kommerziell erhältlich sind.

Die physikalischen Eigenschaften dieser Partikel sind wie folgt:
Durchmesser: 2-4 mm
Dichte:1,1-1,8 g/cm³

Die Bindungseigenschaften dieser Partikel sind bevorzugt wie folgt:
Streptavidin Dichte und Verhalten zur Biotin-Antikörper Bindung sind entsprechend Dynabeads (s.o.).

Vorteile dieser Partikel sind:
- sie sind rot fluoreszierend, dadurch ist die Ablesung erleichtert.
- die Kontrolle der Qualität kann auch in Durchflußzytometrie erfolgen.

Insgesamt sollten die Partikel bevorzugt aus Polystyrene bestehen und einen Durchmesser von 2-4 µm aufweisen. Es ist besonders bevorzugt, wenn diese Mikropartikel auf der Oberfläche Epoxy-, Carboxyl-, und/oder Aminoreste oder Tosylgruppen tragen.

Es ist jedoch dem Fachmann klar, dass die erfindungsgemäß verwendbaren Mikropartikel aus einer großen Vielzahl unterschiedlicher Materialien aufgebaut sein können. Das Gebiet der Mikropartikel-Technologie ist dem Fachmann gut bekannt, und es gibt viele verschiedene kommerziell erhältliche Mikropartikel, die potentiell erfindungsgemäß verwendbar sind. Die Größe der Mikropartikel ist oben beschrieben.

Nach Bindung des Zweitantikörpers an den Bead-Erstantikörper-Antigen-Komplex wird dieser Zweitantikörper nachgewiesen. Hierzu kann entweder dieser Zweitantikörper beispielsweise Fluoreszenz- oder enzymatisch markiert sein, oder es kann auch ein markierter Drittantikörper gegen diesen Zweitantikörper eingesetzt werden. Wird ein Agglutinationstest durchgeführt, ist eine Markierung des Antikörpers nicht nötig.

Solche Tests und Nachweisverfahren sind dem Fachmann gut bekannt. Erfindungsgemäß verwendbar sind Agglutinationstests in einer Gelkarte.

Es ist der vorliegenden Erfindung zu eigen, dass die einzelnen Bestandteile des erfindungsgemäßen Nachweisverfahrens dem Fachmann bekannt sind. Es war jedoch in keiner Weise vorherzusehen, dass eine Kombination dieser einzelnen Schritte zu einem Verfahren führt, dass in so überaus überraschend günstiger Weise für ein solchermaßen intensives Bedürfnis der Fachwelt eine einfache Lösung bietet.

Insbesondere können erfindungsgemäß auch Kits für den Nachweis von Blutzellantigenen bzw. von gegen Blutzellantigene gerichtete Antikörpern bereitgestellt werden, mindestens umfassend kugelförmige Beads, die einer Durchmesser von 2-4 µm und eine Dichte von 1,1-1,8g/cm³ aufweisen, einen spezifischen Antikörper gegen ein Blutzellantigen, sowie eine Gelkarte, die einer Spezifischer anti-human Antikörper Antikörper enthält.

Unter einer weiteren Ausführungsform umfasst der Kit für den Nachweis von Blutzellantigenen mindestens kugelförmige Beads, die einer Durchmesser von 2-4 µm und eine Dichte von 1,1-1,8g/cm³ aufweisen , einen ersten gegen das nachzuweisende Antigen gerichteten Antikörper und eine Gelkarte, die einen zweiten speziesspezifischen Antikörper enthält, der gegen den zweiten gegen das nachzuweisende Antigen gerichteten Antikörper gerichtet ist.
Es ist klar, dass auch weitere Kits, die die in den erfindungsgemäßen Verfahren beschriebenen essentiell benötigten Bestandteile enthalten.

Die nachfolgenden Beispiele erläutern die Erfindung näher. Sie sollen jedoch keineswegs limitierend verstanden werden.

### BEISPIEL 1: Solubilisierung von Zellen

| | |
|---|---|
| Solubilisierungspuffer: | 1,21 g Tris |
| | ad 950 ml NaCl |
| | pH 7,4 |
| | Zugabe von 5 ml Triton X-100 |
| | ad 1000 ml NaCl |

Die Testzellen (Thrombozyten) werden 1 min in einer Tischzentrifuge (Hersteller: Hettich) bei 10000 U/min sedimentiert. Der Überstand wird abgesaugt, und das Zellpellet wird in 100 µl Solubilisierungspuffer aufgenommen.

### VERGLEICHEND BEISPIEL 2

Ein spezifischer anti-GPIIa-IIIb Antikörper (Fa. BioTest) wurde biotinyliert und in Lösung mit Streptavidin beschichteten Beads versetzt. Die Bindung des Antikörpers an die Beads wurde gezeigt. Es hat sich hier gezeigt, dass die Anwesenheit von 1mM Zink die Bindung fördert.

Anschließend werden die Beads mit solubilisierten Blutzellen inkubiert und ein Fluoreszenz-markierter Zweitantikörper hinzugegeben. Ein positiver Testansatz ist gekennzeichnet durch vermehrte Anlagerung des sekundären Antikörpers an den Komplex, was wiederum über die dadurch vermittelte Fluoreszenz des abgetrennten Bead-Erstantikörper-Antigen-Zweitantikörper-Komplexes gemessen werden konnte.

Im Blut konnte das GPIIa-IIIb -Antigen so auf erstaunlich einfache Weise nachgewiesen werden.

### BEISPIEL 3

Statt eines auf Fluoreszenz-Markierung beruhenden Tests wurde ein Agglutinationstest (Gelkartentest, Fa. Diamed, Schweiz) durchgeführt.

Auch auf diese Weise konnte im Blut das GPIIa-IIIb -Antigen so auf erstaunlich einfache Weise nachgewiesen werden.

### VERGLEICHEND BEISPIEL 4

Statt eines Elisa-Tests wurden die mit Zweitantikörper markierten Beads vermittels Durchflußzytometrie nachgewiesen.

Auch auf diese Weise konnte das GIIa-IIIb-Antigen auf erstaunlich einfache Weise nachgewiesen werden.

### BEISPIEL 5

Durchführung eines erfindungsgemäßen Tests unter Verwendung von Streptavidin Partikeln der Firma Diamed.

### Patienten/Spender und Probenmaterial

20 µl Serum (30 min bei 56°C inaktiviert)

### Reagenzien und Geräte

### Rot-fluoreszierende High-Density Streptavidin Polystyrene Partikel (Diamed)

Physikalische Eigenschaften:
Durchmesser: 2-4 µm
Dichte:1,1-1,8 g/cm³

Die Suspension enthält 0,075% Partikel (v/v), daher wurden diese Beads in einem Volumen von 50 µl eingesetzt (im Vergleich Dynabeads 10 µl)

Bindungseigenschaften:
Streptavidin Dichte und Verhalten zur Biotin-Antikörper Bindung sind entsprechend Dynabeads.

Vorteile: Die Partikel sind rot fluoreszierend, dadurch ist die Ablesung erleichtert und die Kontrolle der Qualität kann auch mittels der dem Fachmann gut bekannten Methode der Durchflußzytometrie erfolgen.

Nachteile: Blocken der Partikel nach Coating mit PBS-BSA erscheint notwendig, da eine hohe Absorptiviät dieser Partikel besteht.

| | |
|---|---|
| Puffer | |
| Partikel Puffer | 10 mM PBS-Lösung , pH 7,4, 0,1% Tween 20 |
| PBS-BSA (2%ig): | Dulbecco's Phosphate buffered saline x10 1:10 in aqua dest. verdünnen pH 7,2 Zugabe von 22%igem (1:10)Rinderalbumin |
| Solubilisationspuffer: | 1,21g Tris ad 950 ml NaCl pH 7,4 Zugabe von 5 ml Triton X-100 ad 1000 ml NaCl |

verwendete Antikörper:

| Spezifität | Klon | Spezies | Isotyp | Form | Biotin Überschuss Faktor |
|---|---|---|---|---|---|
| GP IIb/IIIa (CD41) | P2 | Maus | IgG1 κ | purified | 15 |
| GP Ia/IIa (CD49b) | Gi9 | Maus | IgG1 κ | purified | 15 |
| GP Ib/IX (CD 42a) | FMC-25 | Maus | IgG1 κ | purified | 15 |
| GP IV (CD 36) | FA6-152 | Maus | IgG | purified | 15 |
| CD 9 | ALB 6 | Maus | IgG1 κ | purified | 15 |
| Maus IgG Fcγ Frag. | | Ziege | | purified | |
| Human IgG Fcγ Frag. | | Ziege | | Peroxidase | |

| | | | | | |
|---|---|---|---|---|---|
| Biotin: Sulfo-NHS-LC-Biotin, 25 mg, Pierce, Perbio Science, Rockford, USA | | | | | |

Filtration nach Biotinylierung in Millipore Filtereinheiten Ultrafree-MC 30 (Millipore Corporation, Bedford, USA), Filtrationsgrenze 30 kDa Membran: Low binding regenerated Cellulose

### Kalibrierung:

Es werden Spontan-Agglutination getestet und Negativ-Kontrollen mitgeführt. Jede neue Charge oder Kopplung wird quantitativ in einer durchflußzytometrischen Untersuchung kontrolliert. Hierbei werden Antigenbeschichtete Partikel mit einem Fluoreszenz markierten Antikörper, der spezifisch gegen das Antigen gerichtet ist inkubiert. Die durchflußzytometrisch einfach zu messenden Fluoreszenz-Intensität auf der Oberfläche der Partikel ist proportional dem Beschichtungsgrad.

### Qualitätskontrolle: Mitführen von positiven und negativen Kontrollseren

### Vorgehensweise:

### Vorbereitung

### Biotinylierung der monoklonalen Antikörper

Biotin ist ein Vitamin (244 Dalton), dass als Co-Faktor der Carboxylasen fungiert. Durch seine geringe Größe beeinflusst Biotin die Eigenschaften eines Makromoleküls nach Bindung in der Regel nicht. Die reaktive Gruppe für Biotin sind ε-amino-Gruppen von Lysin und Tyrosin (NHS-Biotin) mit der Bildung einer Amin-Bindung. Da Antikörper auch im Bereich der variablen Regionen Lysin enthalten können, kann die Biotinylierung zu einem Verlust der Antigen-Detektion führen. Um diesen Effekt auszuschließen oder zu reduzieren muss für jeden Antikörper eine optimale Biotin-Konzentration (Biotin-Überschuß) gefunden werden, bei der funktionelle Bereiche der Antikörper-Bindung nicht gestört werden (Ternynck and Avrameas 1990). Die Anwendung des NHS-LC-Biotin hat sich für Makromoleküle wie z.B. Glykoproteine als vorteilhaft gezeigt. Hierbei wird durch eine zusätzliche Kette mit 6 Carbonmolekülen (Hexanoat-Spacer, 22 A), eine mögliche sterische Behinderungen der Makromoleküle verhindert. Nach dem Sulfo-NHS-LC-Biotin in Lösung gebracht wurde ist eine schnelle Verarbeitung zu empfehlen, da das gelöste Biotin sehr anfällig für Hydrolyse ist.

### Vorgehensweise:

Das Protein in 1 ml PBS auflösen und mmols des Biotin-Überschußes (Faktor 10-15) berechnen: mmoles Protein x (12 or 20) = mmoles Biotinreagenz-Zugabe: mmoles Biotin zu pipettieren x 556* = mg Biotinreagenz zu geben (* 556 ist das MG des Sulfo-NHS-LC-Biotin)Berechnung: mg Protein/ MG
Protein= mmoles Protein
z.B. 1,3/150 000= 8,7x 10⁻⁶ mmol IgG
8,7x 10⁻⁶ x 20= 1,73x 10⁻⁴ mmol Biotinreagenz
1,73x 10⁻⁴ x 556= 0,096373 mg Biotin (0,1 mg)
1. Das Sulfo-NHS-LC-Biotin vor dem Öffnen Raumtemperatur erreichen lassen.
2. 1 mg Sulfo-NHS-LC-Biotin in 1000 µl dH₂O auflösen (1 µg = 1µl).
3. Inkubation 30 Min Raumtemperatur (alternativ 2 Stunden Inkubation in Eis).
4. Entfernen des nicht konjugierten Biotins durch Zentrifugation mit Millipore Filter MC 30 in PBS.
5. Zentrifugen-Einstellung variiert in Abhängigkeit vom Protein, in der Regel sind 10-15 Minuten bei 10000 U/min. ausreichend, alternativ 30 min bei 3500 U/min
6. Aufbewahren des biotinylierten Antikörper bei 4°C in 0,01 %Natrium Acid.

### Berechnung des Biotinylierungsgrades:

Die Bestimmung des Biotinylierungsgrades erfolgt photometrisch mit Hilfe des HABA Reagenz (2-Hydroxyazobenzene-4-Carboxyl-Säure).
Dazu wird zunächst die Absorption bei 500 nm des HABA Reagenz (360 µl) allein gemessen und notiert, um dann die biotinylierte Probe (40 µl) zuzugeben. 5 Minuten nach der Zugabe wird erneut die Absorption bei 500 nm bestimmt. In Abhängigkeit des Biotin-Gehaltes der Probe findet durch eine kompetitive Bindung im HABA Molekül ein Farbumschlag statt, d.h. je höher ΔA um so höher der Biotinylierungs-Grad der Probe.
1. A500 = (0.9) x (A500 HABA Reagenz) - (A500 des biotinylierten Protein)
2. mmol/ml des biotinylierten Protein = mg biotinylierten Protein/ml der Probe
3. µmoles Biotin/ml Reaktionsmix = A500

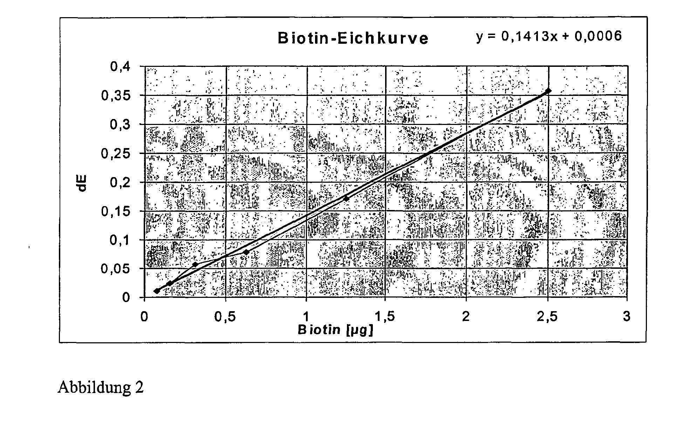

Alternativ zur HABA Reagenztest kann eine Eichkurve der Biotinylierung für jedes verwendete Protein erstellt werden. Nach dieser Kurve kann die optimale Biotin-Menge, die für jedes Protein anders ist, einmalig pro Protein und Charge ermittelt werden.
Eine solche Eichkurve ist in Abbildung 2 gezeigt.
Seren: Kontroll Seren aus -20°C vorsichtig zunächst bei 4°C auftauen, abzentrifugieren (2 min 13.000 U/min; Eppendorfzentrifuge)

Markierung und Solubilisierung der Thrombozyten-Glykoproteine 2 x 10⁷ Test-Zellen (typisiert) 1 min bei 10000 U/min im Eppendorftube abzentrifugieren, Überstand absaugen, Zellpellet in 30 µl PBS-BSA 2% resuspendieren
je Ansatz werden 10 µl glykoproteinspezifischer biotinylierter monoklonaler Antikörper hinzugegeben, für jeden eingesetzten Antikörper muss vorher anhand von Titration nach Biotinylierung eine optimale Verdünnung bestimmt werden (siehe Ausschluss der Spontan-Agglutination) CD 41 1:60, CD 42a und CD49b 1:10, HLA Antikörper 1:200 ! Variationen sind chargenabhängig !
30 Minuten bei 37 °C inkubieren
Testthrombozyten 3 mal mit 100 µl PBS waschen (1 min bei 10000 U/min zentrifugieren, Überstand absaugen und Thrombozytenpellet in 100 µl PBS aufnehmen.
nach dem letzten Waschvorgang die Pellets in je 100 µl Solubilisationspuffer lysieren, einzeln mit der Pipette mischen
30 min bei 4°C inkubieren
Antigen-Isolierung an Streptavidin Polymer-Partikel
Lysate 30 min bei 13000 U/min abzentrifugieren (4°C, Zellmembranreste werden entfernt)
während der Zentrifugation der Lysate die DiaMed Streptavidin Partikel 1 mal mit Partikel-Puffer 1 min bei 10000 U/min waschen
10 µl Partikel (Konzentration 0,5%) zu 70 µl des Thrombozyten-Lysates zugegeben
Inkubation 30 min bei 37 °C
waschen der Partikel 1 x mit 100 µl Partikel-Puffer, 1 min 10000 U/min Überstand absaugen
Partikel sind mit Antigen beschichtet und können nach Resuspension in Partikel
Puffer zu einer Konzentration von 0,075% in das Testsystem eingebracht werden.'

### Testansatz zur Antigen/Antikörper Detektion

20 µl Serum zu dem Partikel-Pellet geben, Pellet gut resuspendieren
Inkubation 30 min bei 37 °C
100 µl Partikel-Puffer zu den Partikel pipettieren
2 mal mit je 100 µl Partikel-Puffer waschen
in 50 µl Partikel Puffer aufnehmen, Pellet vorsichtig mischen
Partikel Suspension direkt auf die Ziege anti-human Karte (Diamed; erläutert in Y. Lapierre et al., (1990), Transfusion 30(2):109-113) pipetieren
Karte ohne weitere Inkubation in DiaMed Karten Zentrifuge bei 900 U/min und 10 min zentrifugieren

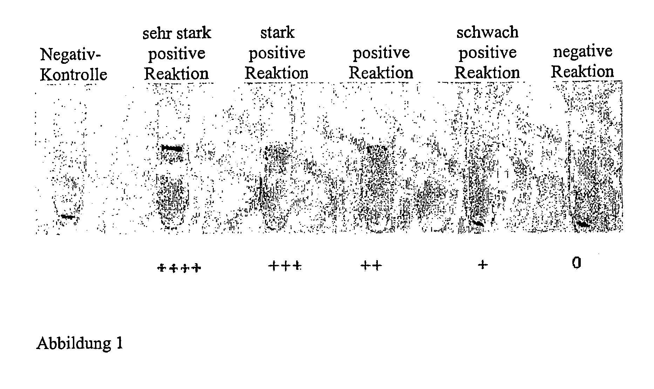

Befund-Beurteilung siehe Abbildung 1.
Man erkennt deutlich die hohe Sensitivität des Tests.

Bisher wurden 34 Seren verschiedener Reaktivität selektiv getestet. Der Vergleich mit dem MAIPA zeigt eine vergleichbare Sensitivität und Spezifität.

Bei N=8 Seren konnte die Spezifität anti- Pla 1 parallel detektier werden
Bei N=2 Seren konnte trotz Vorbefund sowohl in MAIPA als auch im Kartentest kein
anti-Pla 2 detektiert werden
Bei N=4 Seren konnten Antikörper der Spezifität anti Bra nachgewiesen werden
Bei N=4 Seren konnten Autoantikörper verschiedener Spezifität nachgewiesen werden
N=8 Seren wurden negativ getestet in MAIPA und Kartentest
N=8 Seren waren in beiden Tests unspezifisch reagierend

Bei einer Testdauer von 30-45 Minuten (unter Nutzung vorher beschichteter Partikel), ist der Arbeits- und Zeitaufwand minimal.

### Ergebnisdarstellung:

Beurteilung (siehe Abbildung 1)
Positiv: Agglutinierte Beads bilden eine rote Linie auf dem Gel oder sind im Gel verteilt (++++ bis +)
Negativ: Kompaktes Partikelsediment am Boden der Kavität der ID-Karte

## Patentansprüche

1. In vitro-Verfahren zum Nachweis von gegen Blutzell-Antigene gerichteten Antikörpern oder Blutzell-Antigenen in Proben aus Menschen, **dadurch gekennzeichnet, dass** man die nativen gegen Blutzell-Antigene gerichteten Antikörper bzw. die nativen Blutzell-Antigene unter Verwendung von monoklonalen Antikörpern mit Beads, die einen Durchmesser von 2-4 µm und eine Dichte von 1,1-1,8 g/cm3 aufweisen, aus der Probe fischt, und den Nachweis der an die Beads gebundenen, gegen Blutzell-Antigene gerichteten Antikörper bzw. der Blutzell-Antigene durch Agglutination in einer Gelkarte führt, die einen zum Nachweis geeigneten, speziesspezifischen Antikörper enthält, der gegen die Spezies gerichtet ist, aus der der nachzuweisende Antikörper gegen Blutzell-Antigene bzw. der zum Nachweis des Blutzell-Antigens verwendete Antikörper stammt.

2. In vitro-Verfahren zum Nachweis von gegen Blutzell-Antigene gerichteten Antikörpern in Proben aus Menschen nach Anspruch 1, **dadurch gekennzeichnet, dass** man
(i) die Beads mit gegen das spezifische Blutzellantigen gerichteten, nicht-humanen Antikörpern beschichtet,
(ii) zugehörige, spezifische, ausgewählte Blutzell-Antigene, die gegebenenfalls durch Solubilisierung von im Stand der Technik an sich schon bekannten standardisierten Blutzellen oder auch durch rekombinante oder proteinchemische Methoden erhalten werden können, mit den aus (i) erhaltenen beschichteten Beads mischt,
(iii) die entstandenen Komplexe aus mit nicht-humanen Antikörpern beschichteten Beads und den ausgewählten Blutzell-Antigenen mit einer Serumprobe eines zu untersuchenden Patienten mischt, und
(iv) die gegebenenfalls an die Komplexe gebundenen Blutzell-Antikörper des Patienten durch Agglutination in der Gelkarte nachweist.

3. In vitro-Verfahren zum Nachweis von Antikörpern gegen Blutzell-Antigene, nach Anspruch 1, **dadurch gekennzeichnet, dass** man
(i) die Beads mit nicht anti-humanen, speziesspezifischen Antikörpern beschichtet,
(ii) Standardzellen solubilisiert bzw. gentechnisch oder proteinchemisch gewonnene Blutzell-Antigene in Lösung bringt,
(iii) das aus (ii) erhaltene Solubilisat bzw. die Lösung von gentechnisch oder proteinchemisch gewonnenen Blutzell-Antigenen mit spezifischen, gegen das Antigen gerichteten und aus der in (i) definierten Spezies stammenden Antikörpern inkubiert,
(iv) die in (iii) gebildeten Komplexe mit den Beads aus (i) durch Zentrifugation isoliert,
(v) direkt oder später mit aus Patienten stammendem antikörper-haltigem Serum inkubiert , und
(vi) die an die Komplexe gebundenen Antikörper durch Agglutination in der Gelkarte nachweist.

4. In vitro-Verfahren zum Nachweis von Blutzell-Antigenen nach Anspruch 1, **dadurch gekennzeichnet, dass** man
(i) die Beads mit gegen die nachzuweisenden spezifischen Blutzellantigene gerichteten Antikörpern beschichtet,
(ii) Blutproben aus einem zu untersuchenden Patienten mit den darin enthaltenen Blutzellen einer Behandlung unterwirft, durch die die in der Membran der Blutzellen enthaltenen Antigene solubilisiert werden, und gegebenenfalls eine antigenangereicherte Fraktion herstellt,
(iii) die aus (i) erhaltenen beschichteten Beads mit der aus (ii) erhaltenen Probe mischt,
(iv) den Komplex aus den Beads, den gegen die nachzuweisenden spezifischen Blutzell-Antigene gerichteten Antikörpern und den Blutzell-Antigenen mit Antikörpern inkubiert, die spezifisch für das Blutzell-Antigen sind, und
(v) die an die Komplexe gebundenen Antikörper durch Agglutination in der Gelkarte nachweist.

5. In vitro-Verfahren zum Nachweis von Blutzell-Antigenen nach Anspruch 1, **dadurch gekennzeichnet, dass** man
(i) die Beads mit nicht anti-humanen, speziesspezifischen Antikörpern beschichtet,
(ii) Blutzellen aus zu untersuchenden Patienten solubilisiert,
(iii) das aus (ii) erhaltene Solubilisat mit spezifischen, gegen das nachzuweisende Antigen gerichteten und aus der in (i) definierten Spezies stammenden Antikörpern inkubiert,
(iv) die in (iii) gebildeten Komplexe mit den Beads aus (i) durch Zentrifugation isoliert,
(v) die Komplexe mit Antikörpern inkubiert, die gegen das Antigen gerichtet sind, und
(vi) die an die Komplexe gebundenen Antikörper durch Agglutination in der Gelkarte nachweist.

6. In vitro-Verfahren zum Nachweis von Antikörpern gegen Blutzell-Antigene in einer Serumprobe eines Patienten nach Anspruch 1, **dadurch gekennzeichnet, dass** man
(i) biotinylierte, gegen das Antigen gerichtete nicht humane Antikörper in Kontakt bringt mit humanen Testzellen, die die entsprechenden, nativen Blutzell-Antigene tragen,
(ii) den aus (i) gebildeten Komplex Bedingungen unterwirft, unter denen die Blutzellen solubilisieren,
(iii) den Komplex aus Antigen und biotinyliertem Antikörper mit Hilfe der mit Streptavidin oder Avidin beschichteten Beads aus der Probe entfernt,
(iv) den in (iii) gebildeten Komplex mit einer Serumprobe eines zu untersuchenden Patienten in Kontakt bringt, und
(v) die im Falle des Vorhandenseins von spezifischen Antikörpern in der Serumprobe gebildeten Komplexe aus biotinyliertem Antikörper, Antigen und humanem Antikörper aus dem Serum nach Abtrennung des Komplexes aus der Probe durch Agglutination in der Gelkarte nachweist..

7. In vitro-Verfahren zum Nachweis von ausgewählten Blutzell-Antigenen nach Anspruch 1, **dadurch gekennzeichnet, dass** man
(i) biotinylierte, gegen das nachzuweisende Antigen gerichtete Antikörper in Kontakt bringt mit aus der zu untersuchenden Probe stammenden humanen Zellen , die gegebenenfalls die entsprechenden, nativen Blutzell-Antigene tragen,
(ii) den aus (i) gebildeten Komplex Bedingungen unterwirft, unter denen die Blutzellen solubilisieren,
(iii) den Komplex aus Antigen und biotinyliertem Antikörper mit Hilfe der mit Streptavidin oder Avidin beschichteten Beads aus der Probe entfernt,
(iv) den in (iii) gebildeten Komplex mit einem gegen das Antigen gerichteten, bevorzugt markierten Zweitantikörper in Kontakt bringt, und
(v) die gebildeten Komplexe aus biotinyliertem Antikörper, Antigen und Zweitantikörper nach Abtrennung des Komplexes aus der Probe durch Agglutination in der Gelkarte nachweist.

8. Verfahren nach einem der vorstehenden Ansprüche, bei dem fluoreszenzmarkierte oder enzymatisch markierte Antikörper verwendet werden.

9. Verfahren nach einem der vorstehenden Ansprüche, bei dem biotinylierte Antikörper über Streptavidin und/oder Avidin beschichtete Beads gebunden werden.

10. Kit für den Nachweis von gegen Blutzellantigene gerichteten Antikörpern, umfassend mindestens Beads, die einen Durchmesser von 2-4 µm und eine Dichte von 1,1-1,8 g/cm3 aufweisen, einen spezifischen Antikörper gegen ein Blutzellantigen, sowie eine Gelkarte, die einen spezifischen anti-human Antikörper Antikörper enthält.

11. Kit für den Nachweis von Blutzellantigenen , umfassend mindestens Beads, die einen Durchmesser von 2-4 µm und eine Dichte von 1,1-1,8 g/cm3 aufweisen einen ersten gegen das nachzuweisende Antigen gerichteten Antikörper, einen zweiten gegen das nachzuweisende Antigen gerichteten Antikörper und eine Gelkarte, die einen zweiten speziesspezifischen Antikörper enthält, der gegen den zweiten gegen das nachzuweisende Antigen gerichteten Antikörper gerichtet ist.

## Claims

1. In-vitro method of detecting blood cell antigens, or antibodies acting against blood cell antigens, in samples from human beings, **characterised in that** the native blood cell antigens, or the native antibodies acting against blood cell antigens, as the case may be, are fished out of the sample, by using monoclonal antibodies, with beads whose diameter is 2-4 µm and whose density is 1.1 - 1.8 g/cm³, and the detection of the blood cell antigens, or of the antibodies acting against blood cell antigens, is carried out by agglutination in a gel card which contains a species-specific antibody, suitable for the detection, which acts against that species from which the antibody used for detecting the blood cell antigen originates, or from which the antibody acting against blood cell antigens which is to be detected originates.

2. In-vitro method of detecting antibodies acting against blood cell antigens in samples from human beings, according to claim 1, **characterised in that**
(i) the beads are coated with non-human antibodies which act against the specific blood cell antigens,
(ii) associated, specific, selected blood cell antigens, which may, if required, be obtained by solubilising standardised blood cells which were already known per se in the prior art or even by recombinant or protein chemistry methods, are mixed with the coated beads obtained from (i),
(iii) the complexes produced, comprising beads coated with non-human antibodies and the selected blood cell antigens, are mixed with a sample of serum from a patient who is being examined, and
(iv) the patient's blood cell antibodies which may possibly be bound to the complexes are detected by agglutination in the gel card.

3. In-vitro method of detecting antibodies acting against blood cell antigens, according to claim 1, **characterised in that**
(i) the beads are coated with non-anti-human species-specific antibodies,
(ii) standard cells are solubilised or blood cell antigens obtained genetically or by protein chemistry are dissolved into a solution,
(iii) the solubilisate obtained from (ii), or the solution of blood cell antigens obtained genetically or by protein chemistry, as the case may be, is incubated with specific antibodies which act against the antigen and which originate from the species defined in (i),
(iv) the complexes formed in (iii) are isolated, using the beads from (i), by centrifuging,
(v) are incubated with antibody-containing serum originating from the patient immediately or at a later stage, and
(vi) the antibodies bound to the complexes are detected by agglutination in the gel card.

4. In-vitro method of detecting blood cell antigens, according to claim 1, **characterised in that**
(i) the beads are coated with antibodies which act against the specific blood cell antigens which are to be detected,
(ii) samples of blood from a patient who is being examined, and the blood cells contained therein, are subjected to a treatment by which the antigens contained in the membranes of the blood cells are solubilised, and an antigen-enriched fraction is produced if required,
(iii) the coated beads obtained from (i) are mixed with the sample obtained from (ii),
(iv) the complex comprising the beads, the antibodies which act against the specific blood cell antigens which are to be detected, and the blood cell antigens is incubated with antibodies which are specific to the blood cell antigen, and
(v) the antibodies bound to the complexes are detected by agglutination in the gel card.

5. In-vitro method of detecting blood cell antigens, according to claim 1, **characterised in that**
(i) the beads are coated with non-anti-human species-specific antibodies,
(ii) blood cells from the patient who is being examined are solubilised
(iii) the solubilisate obtained from (ii) is incubated with specific antibodies which act against the antigen which is to be detected and which originate from the species defined in (i),
(iv) the complexes formed in (iii) are isolated, using the beads from (i), by centrifuging,
(v) the complexes are incubated with antibodies which act against the antigen, and
(vi) the antibodies bound to the complexes are detected by agglutination in the gel card.

6. In-vitro method of detecting antibodies acting against blood cell antigens in a serum sample from a patient, according to claim 1, **characterised in that**
(i) biotinylated non-human antibodies acting against the antigen are brought into contact with human test cells which carry the appropriate native blood cell antigens,
(ii) the complex formed in (i) is subjected to conditions under which the blood cells solubilise,
(iii) the complex comprising antigens and biotinylated antibodies is removed from the sample by means of beads coated with streptavidin or avidin,
(iv) the complex formed in (iii) is brought into contact with a serum sample from a patient who is being examined, and
(v) the complexes which form in the event of specific antibodies being present in the serum sample, which complexes comprise biotinylated antibodies, antigens and human antibodies from the serum, are detected by agglutination in the gel card once the complexes have been separated out of the sample.

7. In-vitro method of detecting selected blood cell antigens, according to claim 1, **characterised in that**
(i) biotinylated antibodies which act against the antigen to be detected are brought into contact with human cells originating from the specimen being examined, which human cells may possibly carry the appropriate native blood cell antigens,
(ii) the complex formed in (i) is subjected to conditions under which the blood cells solubilise,
(iii) the complex comprising antigens and biotinylated antibodies is removed from the sample by means of the beads coated with streptavidin or avidin,
(iv) the complex formed in (iii) is brought into contact with a second antibody which acts against the antigen, which second antibody is preferably marked, and
(v) the complexes which form comprising biotinylated antibodies, antigens and second antibody are detected by agglutination in the gel card once the complexes have been separated out of the sample.

8. Method according to one of the foregoing claims in which antibodies marked by fluorescence or enzymatically marked antibodies are used.

9. Method according to one of the foregoing claims in which biotinylated antibodies are bound by means of beads coated with streptavidin and/or avidin.

10. Kit for the detection of antibodies acting against blood cell antigens, comprising at least beads whose diameter is 2-4 µm and whose density is 1.1 - 1.8 g/cm³, a specific antibody acting against a blood cell antigen, and a gel card which contains a specific anti-human antibody antibody.

11. Kit for the detection of blood cell antigens, comprising at least beads whose diameter is 2-4 µm and whose density is 1.1 - 1.8 g/cm³, a first antibody acting against the antigen to be detected, a second antibody acting against the antigen to be detected, and a gel card which contains a second species-specific antibody which acts against the second antibody acting against the antigen to be detected.

## Revendications

1. Procédé in vitro de détection d'anticorps dirigés contre des antigènes de cellules sanguines ou d'antigènes de cellules sanguines dans des échantillons provenant d'humains, **caractérisé en ce qu'**on capture, à partir de l'échantillon, respectivement les anticorps dirigés contre les antigènes de cellules sanguines natifs ou les antigènes de cellules sanguines natifs en utilisant des anticorps monoclonaux fixés sur des billes, qui présentent un diamètre de 2-4 µm et une masse volumique de 1,1-1,8 g/cm³, et **en ce qu'**on réalise la détection respectivement des anticorps dirigés contre les antigènes de cellules sanguines ou des antigènes de cellules sanguines, liés aux billes, par agglutination sur une carte gel, qui contient un anticorps espèce spécifique approprié pour la détection, qui est dirigé contre l'espèce de laquelle provient respectivement l'anticorps contre les antigènes de cellules sanguines à détecter ou l'anticorps utilisé pour la détection de l'antigène de cellule sanguine.

2. Procédé in vitro pour la détection d'anticorps dirigés contre des antigènes de cellules sanguines dans des échantillons provenant d'humains selon la revendication 1, **caractérisé en ce que**
(i) les billes sont enrobées d'anticorps non humains dirigés contre un antigène de cellule sanguine spécifique,
(ii) on mélange des antigènes de cellules sanguines sélectionnés, spécifiques, appropriés, qui peuvent être éventuellement obtenus par solubilisation de cellules sanguines standardisées déjà connues dans l'état de la technique ou bien par des méthodes recombinantes ou de chimie protéique, avec les billes enrobées obtenues au paragraphe (i),
(iii) on mélange les complexes obtenus constitués de billes enrobées d'anticorps non humains et d'antigènes de cellules sanguines sélectionnés avec un échantillon sérique d'un patient à examiner, et
(iv) on détecte les anticorps de cellule sanguine du patient liés éventuellement aux complexes par agglutination dans la carte gel.

3. Procédé in vitro pour la détection d'anticorps contre des antigènes de cellule sanguine, selon la revendication 1, **caractérisé en ce que**
(i) on enrobe les billes avec des anticorps spécifiques non antihumains,
(ii) on solubilise des cellules standard ou on met en solution des antigènes de cellule sanguine obtenus par technique génétique ou chimie protéique,
(iii) on incube le solubilisé obtenu en (ii) ou la solution d'antigènes de cellule sanguine obtenus par chimie protéique ou technique génétique avec des anticorps spécifiques provenant des espèces définies en (i) dirigés contre l'antigène,
(iv) on isole les complexes formés en (iii) avec les billes issues de (i) par centrifugation,
(v) on procède à une incubation directement ou plus tard avec un sérum contenant des anticorps provenant de patients, et
(vi) on détecte les anticorps liés aux complexes par agglutination dans la carte gel.

4. Procédé in vitro pour la détection d'antigènes de cellule sanguine selon la revendication 1, **caractérisé en ce que**
(i) on enrobe les billes avec des anticorps dirigés contre les antigènes de cellule sanguine spécifiques à détecter,
(ii) on soumet des échantillons sanguins issus d'un patient à examiner avec les cellules sanguines contenues dedans à un traitement, grâce auquel les antigènes contenus dans les membranes des cellules sanguines sont solubilisés, et on fabrique éventuellement une fraction enrichie en antigènes,
(iii) on mélange les billes enrobées obtenues en (i) avec celles obtenues en (ii),
(iv) on incube le complexe constitué des billes, les anticorps dirigés contre les antigènes de cellule sanguine spécifiques à détecter et les antigènes de cellule sanguine avec les anticorps, qui sont spécifiques à l'antigène de cellule sanguine, et
(v) on détecte les anticorps liés aux complexes par agglutination dans la carte gel.

5. Procédé in vitro pour la détection d'antigènes de cellule sanguine, selon la revendication 1, **caractérisé en ce que**
(i) on enrobe les billes avec des anticorps spécifiques à certaines espèces et non antihumains,
(ii) on solubilise des cellules sanguines issues d'un patient à examiner,
(iii) on incube le solubilisé obtenu en (ii) avec des anticorps spécifiques provenant des espèces définies en (i) et dirigés contre l'antigène à détecter,
(iv) on isole les complexes formés en (iii) avec les billes issues de (i) par centrifugation,
(v) on procède à une incubation des complexes avec des anticorps, qui sont dirigés contre l'antigène, et
(vi) on détecte les anticorps liés aux complexes par agglutination dans la carte gel.

6. Procédé in vitro de détection d'anticorps contre des antigènes de cellule sanguine dans un échantillon sérique d'un patient selon la revendication 1, **caractérisé en ce que**
(i) on met en contact des anticorps non humains dirigés contre l'antigène avec des cellules tests humaines, qui portent les antigènes de cellule sanguine natifs correspondants,
(ii) on place le complexe formé en (i) dans des conditions, dans lesquelles les cellules sanguines se solubilisent,
(iii) on élimine de l'échantillon le complexe constitué de l'antigène et d'anticorps biotinylés à l'aide des billes enrobées de streptavidine ou d'avidine,
(iv) on met en contact le complexe formé en (iii) avec l'échantillon sérique d'un patient à examiner, et
(v) on détecte des complexes formés à partir d'anticorps biotinylés, de l'antigène et d'anticorps humains provenant du sérum après isolement du complexe de l'échantillon par agglutination dans la carte gel, dans le cas où des anticorps spécifiques sont présents dans l'échantillon sérique.

7. Procédé in vitro pour la détection d'antigènes de cellule sanguine sélectionnés selon la revendication 1, **caractérisé en ce que**
(i) on met en contact des anticorps biotinylés dirigés contre l'antigène à détecter avec des cellules humaines provenant de l'échantillon à étudier, qui portent éventuellement les antigènes de cellule sanguine natifs correspondants,
(ii) on place le complexe formé en (i) dans des conditions, dans lesquelles les cellules sanguines se solubilisent,
(iii) on élimine de l'échantillon le complexe constitué de l'antigène et d'anticorps biotinylés à l'aide des billes enrobées de streptavidine ou d'avidine,
(iv) on met en contact le complexe formé en (iii) avec un deuxième anticorps dirigé contre l'antigène, marqué de préférence, et
(v) on détecte des complexes formés à partir d'anticorps biotinylés, de l'antigène et du deuxième anticorps après isolement du complexe de l'échantillon par agglutination dans la carte gel.

8. Procédé selon l'une quelconque des revendications précédentes, dans lequel on utilise des anticorps marqués de façon enzymatique ou par fluorescence.

9. Procédé selon l'une quelconque des revendications précédentes, dans lequel des anticorps biotinylés sont liés par des billes enrobées de streptavidine et/ou d'avidine.

10. Kit de détection d'anticorps dirigés contre des antigènes de cellule sanguine, comprenant au moins des billes, qui présentent un diamètre de 2-4 µm et une masse volumique de 1,1-1,8 g/cm³, un anticorps spécifique contre un antigène de cellule sanguine, ainsi qu'une carte gel, qui contient des anticorps anticorps antihumain spécifiques.

11. Kit de détection d'antigènes de cellule sanguine, comprenant au moins des billes, qui présentent un diamètre de 2-4 µm et une masse volumique de 1,1-1,8 g/cm³, un premier anticorps dirigé contre l'antigène à détecter, un deuxième anticorps dirigé contre l'antigène à détecter et une carte gel, qui contient un deuxième anticorps espèce spécifique, qui est dirigé contre le deuxième anticorps dirigé contre l'antigène à détecter.
